# FASCICULE DE BREVET EUROPEEN

(11) **EP 1 064 922 B1**
(45) Date de publication et mention de la délivrance du brevet: **20.07.2005**
(21) Numéro de dépôt: 00401363.7
(22) Date de dépôt: 18.05.2000
(51) Int. Cl.: A61K 7/42

(54) **Compositions photoprotectrices contenant un composé bis-hydroxyphenylbenzotriazole et un composé à groupements benzoazolyle ou benzodiazolyle**
Lichtschutzmittel enthaltend eine bis-Hydroxyphenylbenzotriazol Verbindung und eine Verbindung mit Benzoazolyl- oder Benzodiazolylgruppen
Photoprotective compositions containing a bis-hydroxyphenylbenzotriazole compound and a compound having benzoazolyl or benzodiazolyl groups

(30) Priorité: 08.06.1999 FR 9907205
(43) Date de publication de la demande: 03.01.2001
(73) Titulaire: L'OREAL, 75008 Paris (FR)
(72) Inventeur: Candau, Didier, 91570 Bievres (FR)
(74) Mandataire: Miszputen, Laurent

(56) Documents cités:
- EP-A- 0 893 119

## Description

La présente invention concerne de nouvelles compositions cosmétiques à usage topique plus particulièrement destinées à la photoprotection de la peau et/ou des cheveux contre le rayonnement ultraviolet (compositions ci-après dénommées plus simplement compositions antisolaires), ainsi que leur utilisation dans l'application cosmétique susmentionnée. Plus précisément encore, elle concerne des compositions antisolaires comprenant, dans un support cosmétiquement acceptable, une association entre (a) au moins un composé hydrocarboné bis-hydroxyphénylbenzotriazole selon la revendication 1 à titre de premier filtre et (b), à titre de deuxième filtre, au moins un composé comportant au moins deux groupes benzoazolyle **selon la revendication 1 ;** lesdits premier et second filtres étant présents dans lesdites compositions dans une proportion produisant une activité synergique au niveau des indices de protection solaire conférés.

On sait que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel ; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions photo toxiques ou photo allergiques. Il est donc souhaitable de filtrer aussi le rayonnement UV-A.

De nombreuses compositions cosmétiques destinées à la photoprotection (UV-A et/ou UV-B) de la peau ont été proposées à ce jour.

Ces compositions antisolaires se présentent assez souvent sous la forme d'une émulsion de type huile-dans-eau (c'est à dire un support cosmétiquement acceptable constitué d'une phase continue dispersante aqueuse et d'une phase discontinue dispersée huileuse) qui contient, à des concentrations diverses, un ou plusieurs filtres organiques classiques, lipophiles et/ou hydrophiles, capables d'absorber sélectivement les rayonnements UV nocifs, ces filtres (et leurs quantités) étant sélectionnés en fonction de l'indice de protection recherché (l'indice de protection (IP) s'exprimant mathématiquement par le rapport du temps d'irradiation nécessaire pour atteindre le seuil érythématogène avec le filtre UV au temps nécessaire pour atteindre le seuil érythématogène sans filtre UV).

Or, à la suite d'importantes recherches menées dans le domaine de la photoprotection évoqué ci-dessus, la Demanderesse a découvert, de façon inattendue et surprenante, que la combinaison, dans des proportions comprises dans des limites bien déterminées, de deux filtres solaires particuliers et déjà connus en soi dans l'état de l'art, permettait, du fait d'un effet de synergie remarquable, d'obtenir des compositions antisolaires présentant des indices de protection nettement améliorés, et en tous cas largement supérieurs à ceux qui peuvent être obtenus soit avec l'un ou l'autre des filtres utilisé seul.

Cette découverte est à la base de la présente invention.

Ainsi, conformément à l'un des objets de la présente invention, il est maintenant proposé de nouvelles compositions cosmétiques, en particulier antisolaires, qui sont essentiellement caractérisées par le fait qu'elles comprennent, dans un support cosmétiquement acceptable, (i) un composé hydrocarboné bis-hydroxyphénylbenzotriazole selon la revendication 1, à titre de premier filtre, et (ii) au moins un composé comportant par molécule au moins deux groupes benzazolyle **selon la revendication 1,** à titre de second filtre ; lesdits premier et second filtres étant présents dans lesdites compositions dans une proportion produisant une activité synergique au niveau des indices de protection solaire conférés.

La présente invention a également pour objet l'utilisation de telles compositions pour la fabrication de compositions cosmétiques destinées à la protection de la peau et/ou des cheveux contre le rayonnement ultraviolet, en particulier le rayonnement solaire.

D'autres caractéristiques, aspects et avantages de la présente invention apparaîtront à la lecture de la description détaillée qui va suivre.

Comme composés hydrocarbonés bis-hydroxyphénylbenzotriazole conformes à l'invention, on utilisera le composé répondant à la formule suivante

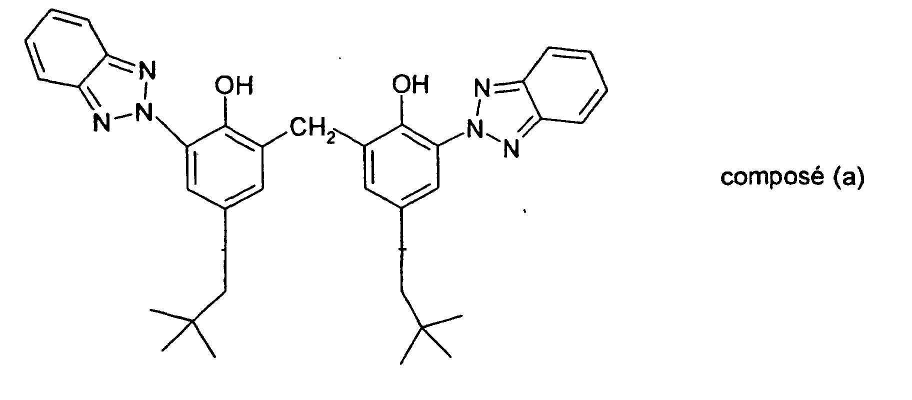

Le filtre du type composé hydrocarboné bis-hydroxyphénylbenzotriazole selon l'invention est généralement présents dans les compositions filtrantes selon l'invention à une concentration totale comprise entre 0,1 et 15 % en poids environ, et de préférence entre 0,2 et 10 % en poids environ, par rapport au poids total de la composition.

Selon une forme particulière de l'invention, les dérivés hydrocarbonés bis-hydroxyphénylbenzotriazole peut se présenter sous une forme particulaire et être insolubles ou substantiellement insolubles dans les solvants usuels cosmétiques (eau, alcools, corps gras).

Par composés insolubles ou substantiellement insolubles, on entend, au sens de la présente invention, des composés dont la solubilité dans l'eau est inférieure à 0,1 % en poids, dont la solubilité dans l'huile de vaseline est inférieure à 1% en poids, et enfin, dont la solubilité dans un mélange d'esters de triglycérides tel que le « Miglyol 812 » commercialisé par la société Dynamit Nobel est inférieure à 2%, également en poids.

Les composés bis-hydroxyphénylbenzotriazole peuvent être amenés sous une forme particulaire convenable par tout moyen ad-hoc tel que notamment broyage à sec ou en milieu solvant, tamisage, atomisation, micronisation, pulvérisation.

Selon un mode particulier de l'invention, les composés bis-hydroxyphénylbenzotriazole peuvent se présenter sous forme insoluble micronisée. La taille moyenne de telles particules variera en général de 0,01 à 2µm et plus préférentiellement de 0,02 à 1,5 µm et plus particulièrement de 0,05 à 1,0 µm.

Les composés bis-hydroxyphénylbenzotriazole sous forme micronisée peut être obtenu par un procédé de broyage d'un dérivé hydrocarboné d'hydroxyphénylbenzotriazole sous forme de particules de taille grossière en présence d'un tensio-actif approprié permettant d'améliorer la dispersion des particules ainsi obtenues dans les formulations cosmétiques.

Un exemple de procédé de micronisation de composés bis-hydroxyphénylbenzotriazole est décrit dans les demandes GB-A-2 303 549 et EP-A-893 119. L'appareil de broyage utilisé selon ces documents peut être un broyeur à jet, à billes, à vibration ou à marteau et de préférence un broyeur à haute vitesse d'agitation ou un broyeur à impact et plus particulièrement un broyeur à billes rotatives, un broyeur vibrant, à broyeur à tube ou un broyeur à tige.

Selon ce procédé particulier, on utilise à titre de tensio-actifs pour le broyage desdits composés bis-hydroxyphénylbenzotriazole, les alkylpolyglucosides de structure CₙH₂ₙ₊₁ O(C₆H₁₀O₅)ₓH dans laquelle n est un entier de 8 à 16 et x est le degré moyen de polymérisation de l'unité (C₆H₁₀O₅) et varie de 1,4 à 1,6. Ils peuvent être choisis parmi des esters en C₁-C₁₂ d'un composé de structure CₙH₂ₙ₊₁ O(C₆H₁₀O₅)ₓH et plus précisément un ester obtenu par réaction d'un acide carboxylique en C₁-C₁₂ tel que l'acide formique, acétique, propionique, butyrique, sulfosuccinique, citrique ou tartrique avec une ou plusieurs fonctions OH libres sur l'unité glucoside (C₆H₁₀O₅). Lesdits tensio-actifs sont utilisés en général à une concentration de allant de 1 à 50% en poids et plus préférentiellement de 5 à 40% en poids par rapport au filtre hydrocarboné bis-hydroxyphénylbenzotriazole dans sa forme micronisée.

le composé comportant au moins deux groupes benzoazolyle conforme à l'invention est un filtre UV-A hydrosolubles déjà connus dans la demande de brevet EP-A-0669323. Il est décrit et préparé selon les synthèses indiquées dans le brevet US 2,463,264 ainsi que la demande de brevet EP-A-0669323.

Comme composé comportant au moins deux groupes benzoazolyle conforme à l'invention, on utilisera un sel de l'acide 1,4-bis-benzimidazolyl-phènylèn-3,3',5,5'-tétrasulfonique de structure suivante:

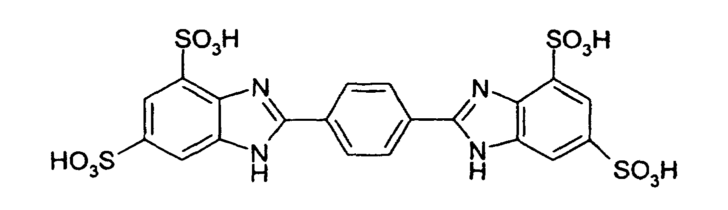

Le composé à groupements benzoazolyle conformes à l'invention peut être présents dans les compositions selon l'invention à une concentration comprise entre 0,1 et 15%, de préférence entre 0,2 et 10%, en poids par rapport au poids total de la composition.

Comme indiqué précédemment, selon une caractéristique essentielle de la présente invention, il convient que les deux types de filtres solaires soient tous deux présents dans la composition finale dans une proportion respective telle qu'un effet de synergie au niveau de l'indice de protection conféré par l'association résultante, soit obtenu de manière notable, substantielle et significative.

En outre, et d'une manière générale, on notera que les concentrations et rapports en composés bis-hydroxyphénylbenzotriazole et en composés à groupements benzoazolyle tels que définis précédemment sont choisis de manière telle que l'indice de protection solaire de la composition finale soit de préférence d'au moins 2.

Selon un mode préféré de réalisation de la présente invention, le support cosmétiquement acceptable dans lequel se trouvent contenus les différents types de filtres est une émulsion de type huile-dans-eau.

Les compositions cosmétiques antisolaires selon l'invention peuvent bien entendu contenir un ou plusieurs filtres solaires complémentaires actifs dans l'UVA et/ou l'UVB (absorbeurs), hydrophiles ou lipophiles, autres bien sûr que les deux filtres mentionnés ci-avant. Ces filtres complémentaires peuvent être notamment choisis parmi les dérivés cinnamiques ; les dérivés salicyliques, les dérivés du camphre ; les dérivés de triazine tels que ceux décrits dans les demandes de brevet EP863145, EP517104, EP570838, EP796851, EP775698 et EP878469 ; les dérivés de la benzophénone ; les dérivés du dibenzoylméthane ; les dérivés de β, β'-diphénylacrylate, les dérivés de benzimidazole ; les dérivés de l'acide p-aminobenzoïque ; les polymères filtres et silicones filtres tels que ceux décrits dans la demande WO-93/04665.

Comme exemples de filtres solaires complémentaires actifs dans l'UV-A et/ou l'UV-B, on peut citer :
l'acide p-aminobenzoïque,
le p-aminobenzoate oxyéthyléné (25mol),
le p-diméthylaminobenzoate de 2-éthylhexyle,
le p-aminobenzoate d'éthyle N-oxypropyléné
le p-aminobenzoate de glycérol,
le salicylate d'homomenthyle,
le salicylate de 2-éthylhexyle,
le salicylate de triéthanolamine,
le salicylate de 4-isopropylbenzyle,
le 4-ter-butyl-4'-méthoxy-dibenzoylméthane,
le 4-isopropyl-dibenzoylméthane,
le 4-méthoxy cinnamate de 2-éthylhexyle,
le diisopropyl cinnamate de méthyle,
le 4-méthoxy cinnamate d'isoamyle,
le 4-méthoxy cinnamate de diéthanolamine,
l'anthranilate de menthyle,
le 2-éthylhexyl-2-cyano-3,3'-diphénylacrylate,
l'éthyl-2-cyano-3,3'-diphénylacrylate,
l'acide 2-phényl benzimidazole 5-sulfonique et ses sels,
le 3-(4'-triméthylammonium)-benzylidèn-bornan-2-on-méthylsulfate,
le 2-hydroxy-4-méthoxybenzophénone,
le 2-hydroxy-4-méthoxybenzophénone-5-sulfonate,
le 2,4-dihydroxybenzophénone,
le 2,2',4,4'-tétrahydroxybenzophénone,
le 2,2'-dihydroxy-4,4'diméthoxybenzophénone,
le 2-hydroxy-4-n-octoxybenzophénone,
le 2-hydroxy-4-méthoxy-4'-méthylbenzophénone,
l'acide a-(2-oxoborn-3-ylidène)-tolyl-4-sulfonique et ses sels
le 3-(4'-sulfo)benzylidèn-bornan-2-one et ses sels,
le 3-(4'méthylbenzylidène)-d,l-camphre,
le 3-benzylidène-d,l-camphre,
l'acide benzène 1,4-di(3-méthylidène-10-camphosulfonique) et ses sels, l'acide urocanique,
la 2,4,6-tris-[p-(2'-éthylhexyl-1'-oxycarbonyl)anilino]-1,3,5-triazine, 2-[(p-(tertiobutylamido)anilino]-4,6-bis-[(p-(2'-éthylhexyl-1'-oxycarbonyl)anilino]-1,3,5-triazine,
la 2,4-bis {[4-2-éthyl-hexyloxy)]-2-hydroxy]-phenyl}-6-(4-méthoxy-phenyl)-1,3,5-triazine ;
le polymère de N-(2 et 4)-[(2-oxoborn-3-ylidèn)méthyl] benzyl]-acrylamide, les polyorganosiloxanes à fonction malonate.

Les compositions selon l'invention peuvent également contenir des agents de bronzage et/ou de brunissage artificiels de la peau (agents autobronzants), tels que par exemple de la dihydroxyacétone (DHA).

Les compositions cosmétiques selon l'invention peuvent encore contenir des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A-0518772 et EP-A-0518773.

Les compositions de l'invention peuvent comprendre en outre des adjuvants cosmétiques classiques notamment choisis parmi les corps gras, les solvants organiques, les épaississants, les adoucissants, les antioxydants, les opacifiants, les stabilisants, les émollients, les hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants, ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les poly-α-oléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs.

Les épaississants peuvent être choisis notamment parmi les homopolymères d'acide acrylique réticulés, les gommes de guar et celluloses modifiées ou non telles que la gomme de guar hydroxypropylée, la méthylhydroxyéthylcellulose, l'hydroxypropylméthyl cellulose ou encore l'hydroxyéthylcellulose.

Bien entendu, l'homme de l'art veillera à choisir ce ou ces éventuels composés complémentaires et/ou leurs quantités de manière telle que les propriétés avantageuses, en particulier le niveau de photoprotection, attachées intrinsèquement à l'association binaire conforme à l'invention ne soient pas, ou substantiellement pas, altérées par la ou les adjonctions envisagées.

Les compositions de l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR2315991 et FR2416008).

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de poudre, de bâtonnet solide, de stick, de mousse aérosol ou de spray.

Lorsque la composition cosmétique selon l'invention est utilisée pour la protection des cheveux, elle peut se présenter sous forme de shampooing, de lotion, de gel, d'émulsion, de dispersion vésiculaire non ionique et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation, et le ou les (co)émulsionnant(s) de 0,5 à 20% en poids, de préférence de 2 à 10% en poids, par rapport à l'ensemble de la formulation.

Comme indiqué en début de description, un autre objet de la présente invention réside dans un procédé de traitement cosmétique de la peau ou des cheveux destiné à les protéger contre les effets des rayons UV consistant à appliquer sur ceux-ci une quantité efficace d'une composition cosmétique telle que définie ci-dessus.

Des exemples concrets, mais nullement limitatifs, illustrant l'invention, vont maintenant être donnés.

### EXEMPLE

| **COMPOSITION** | **Quantité** |
|---|---|
| Mélange d'alcool cétylstéarylique et d'alcool cétylstéarylique oxyéthyléné (33 OE) 80/20 (SINNOWAX AO -HENKEL) | 7 g |
| Mélange de mono et distéarate de glycérol (CERASYNT SD-V ISP) | 2 g |
| Alcool cétylique | 1.5 g |
| Polydiméthyl siloxane (DOW CORNING 200 FLUID -DOW CORNING) | 1 g |
| Benzoate d'alcools en C₁₂/C₁₅ (WITCONOL TN -WITCO) | 15 g |
| Glycérine | 20 g |
| Sel de sodium de l'acide 1,4-bis-benzimidazolyl-phènylèn-3,3',5,5'-tétrasulfonique (composé 4) | 3 g |
| 2,2'-méthylène-bis-[6-(2H-benzotriazol-2-yl)-4-(1,1,3,3-tetraméthylbutyl)phénol] (composé (a)) vendu sous le nom MIXXIM BB/100 par le société FAIRMOUNT CHEMICAL | 5 g |
| Triéthanolamine | qs pH 7 |
| Conservateurs | qs |
| Eau déminéralisée qsp | 100 g |

## Revendications

1. Composition cosmétique à usage topique, en particulier pour la photoprotection de la peau et/ou des cheveux, **caractérisée par le fait qu'**elle comprend, dans un support cosmétiquement acceptable:
(a) à titre de premier filtre, le composé hydrocarboné bis-hydroxyphénylbenzotriazole répondant à la formule suivante **:** 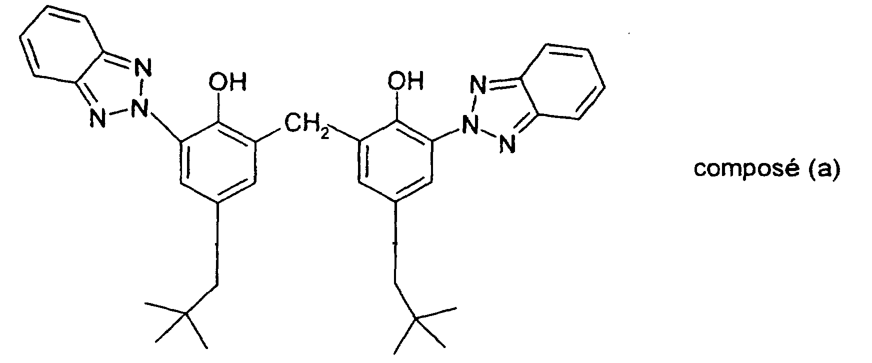
et (b) à titre de deuxième filtre, au moins un le composé comportant au moins deux groupes benzoazolyle constitué par un sel de l'acide 1,4-bis-benzimidazolyl-phènylèn-3,3',5,5'-tétrasulfonique de structure **:** 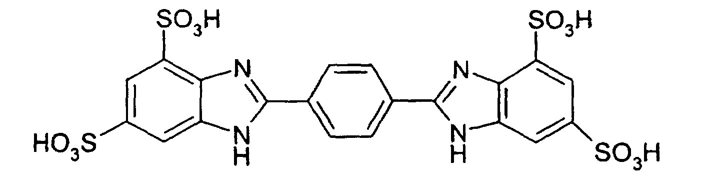
lesdits premier et second filtres étant présents dans une proportion produisant une activité synergique au niveau des indices de protection solaire conférés.

2. Composition selon la revendication 1, **caractérisée par le fait que** le composé hydrocarboné bis-hydroxyphénylbenzotriazole est présents à une concentration allant de 0,1 à 15 % en poids environ par rapport au poids total de la composition.

3. Composition selon la revendication **2**, **caractérisée par le fait que** le composé hydrocarboné bis-hydroxyphénylbenzotriazole est présent à une concentration allant 0,2 et 10 % en poids environ par rapport au poids total de la composition.

4. Composition selon l'une quelconque des revendications 1 à 3, **caractérisée par le fait que** le composé hydrocarboné d'hydroxyphénylbenzotriazole se présente sous forme de particules insolubles.

5. Composition selon l'une quelconque des revendications 1 à 4, **caractérisée par le fait que** le composé hydrocarboné d'hydroxyphénylbenzotriazole se présente sous forme insoluble micronisée

6. Composition selon la revendication 5 où le composé hydrocarboné bis-hydroxyphénylbenzotriazole sous forme insoluble micronisée a une taille moyenne de particule allant de 0,01 à 2µm

7. Composition selon la revendication 6, où le composé hydrocarboné bis-hydroxyphénylbenzotriazole sous forme insoluble micronisée a une taille moyenne de particule allant de 0,02 à 1,5 µm.

8. Composition selon la revendication 7, où le composé hydrocarboné bis-hydroxyphénylbenzotriazole sous forme insoluble micronisée a une taille moyenne de particule allant de 0,05 à 1,0 µm.

9. Composition selon l'une quelconque des revendications 5 à 8, **caractérisée par le fait que** le ou les dérivés hydrocarbonés d'hydroxyphénylbenzotriazole sous forme insoluble micronisée sont susceptibles d'être obtenus par un procédé de broyage d'un dérivé hydrocarboné d'hydroxyphénylbenzotriazole sous forme de particules de taille grossière en présence d'un tensio-actif.

10. Composition selon la revendication 9 , où le tensio-actif est choisi parmi les alkylpolyglucosides de structure CₙH₂ₙ₊₁ O(C₆H₁₀O₅)ₓH dans laquelle n est un entier de 8 à 16 et x est le degré moyen de polymérisation de l'unité (C₆H₁₀O₅) et varie de 1,4 à 1,6.

11. Composition selon la revendication 10, où le tensio-actif alkylpolyglucoside est susceptible d'être obtenu par réaction d'un acide carboxylique en C₁-C₁₂ avec une ou plusieurs fonctions OH libres sur l'unité glucoside (C₆H₁₀O₅).

12. Composition selon la revendication 11, où ledit acide carboxylique en C₁-C₁₂ est choisi parmi l'acide formique, l'acide acétique, l'acide propionique, l'acide butyrique, l'acide sulfosuccinique, l'acide tartrique ou l'acide citrique.

13. Composition selon l'une quelconque des revendications 9 à 12, **caractérisée par le fait que** le tensio-actif est utilisé à une concentration allant de 1 à 50% en poids par rapport au filtre dérivé hydrocarboné d'hydroxyphénylbenzotriazole dans sa forme micronisée.

14. Composition selon l'une quelconque des revendications 1 à 13, **caractérisée par le fait que** le composé à groupements benzoazolyle est présent à une concentration comprise entre 0,1 et 15 % en poids par rapport au poids total de la composition.

15. Composition selon la revendication 14, **caractérisée par le fait que** ladite concentration est comprise entre 0,2 et 10 % en poids par rapport au poids total de la composition.

16. Composition selon l'une quelconque des revendications 1 à 15, **caractérisée par le fait que** ledit support cosmétiquement acceptable se présente sous la forme d'une émulsion de type huile-dans-eau.

17. Composition selon l'une quelconque des revendications 1 à 16, **caractérisée par le fait qu'**elle comprend en outre un ou plusieurs filtres solaires complémentaires actifs dans l'UV-A et/ou UV-B, hydrophiles ou lipophiles, différents desdits premier et deuxième filtres.

18. Composition selon la revendication 17, **caractérisée par le fait que** lesdits filtres organiques complémentaires sont choisis parmi les dérivés cinnamiques, les dérivés salicyliques, les dérivés du camphre, les dérivés de triazine, les dérivés de benzimidazole autres que ceux définis dans les revendications précédentes, les dérivés de la benzophénone, les dérivés du dibenzoylméthane, les dérivés de β,β'-diphénylacrylate, les dérivés de l'acide p-aminobenzoïque, les polymères filtres et silicones filtres.

19. Composition selon l'une quelconque des revendications 1 à 18 **caractérisée par le fait qu'**elle comprend en outre, **à** titre d'agents photoprotecteurs UV complémentaires, des pigments ou des nanopigments d'oxydes métalliques, enrobés ou non.

20. Composition selon la revendication **19**, **caractérisée par le fait que** lesdits pigments ou nanopigments sont choisis parmi les oxydes de titane, de zinc, de fer, de zirconium, de cérium, enrobés ou non.

21. Composition selon l'une quelconque des revendications 1 à 20, **caractérisée par le fait qu'**elle comprend en outre au moins un agent de bronzage et/ou de brunissage artificiel de la peau.

22. Composition selon l'une quelconque des revendications **1 à 21**, **caractérisée par le fait qu'**elle comprend en outre au moins un adjuvant choisi parmi les corps gras, les solvants organiques, les épaississants, les adoucissants, les antioxydants, les opacifiants, les stabilisants, les émollients, les hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants.

23. Composition selon l'une quelconque des revendications 1 à 22 , **caractérisée par le fait qu'**il s'agit d'une composition protectrice de l'épiderme humain ou d'une composition antisolaire et qu'elle se présente sous forme d'une dispersion vésiculaire non ionique, d'une émulsion, en particulier d'une émulsion de type huile-dans-eau, d'une crème, d'un lait, d'un gel, d'un gel crème, d'une suspension, d'une dispersion, d'une poudre, d'un bâtonnet solide, d'une mousse ou d'un spray.

24. Composition selon l'une quelconque des revendications 1 à 22, **caractérisée par le fait qu'**il s'agit d'une composition de maquillage des cils, des sourcils ou de la peau et qu'elle se présente sous forme solide ou pâteuse, anhydre ou aqueuse, d'une émulsion, d'une suspension ou d'une dispersion.

25. Composition selon l'une quelconque des revendications 1 à 22, **caractérisée par le fait qu'**il s'agit d'une composition destinée à la protection des cheveux contre les rayons ultraviolets et qu'elle se présente sous la forme d'un shampooing, d'une lotion, d'un gel, d'une émulsion, d'une dispersion vésiculaire non ionique.

26. Utilisation de la composition définie à l'une quelconque des revendications 1 à 22 pour la fabrication de compositions cosmétiques pour la protection de la peau et/ou des cheveux contre le rayonnement ultraviolet, en particulier le rayonnement solaire.

## Patentansprüche

1. Kosmetische Zusammensetzung zur topischen Anwendung und insbesondere für den Lichtschutz der Haut und/ oder der Haare, **dadurch gekennzeichnet, dass** in einem kosmetisch akzeptablen Träger enthalt:
(a) das Bis-hydroxyphenylbenzotriazol mit Kohlenwasserstoffgruppe der folgenden Formel: 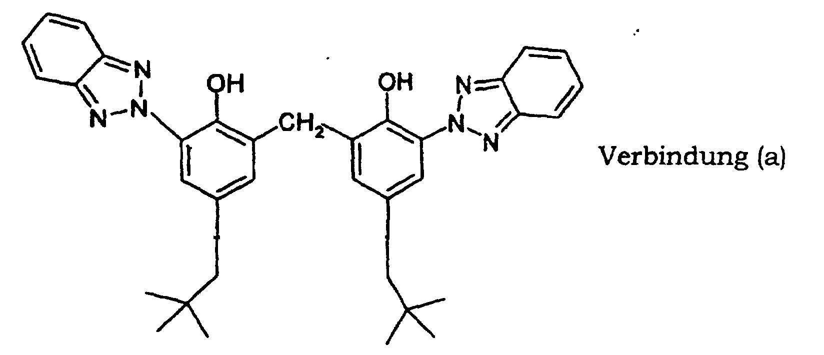 als erstes Filter und
(b) die Verbindung mit mindestens zwei Benzoazolylgruppen, bei der es sich um ein Salz der 1,4-Bis-benzimidazolyl-phenylen-3,3',5,5'-tetrasulfonsäure der folgenden Struktur handelt, als zweites Filter: 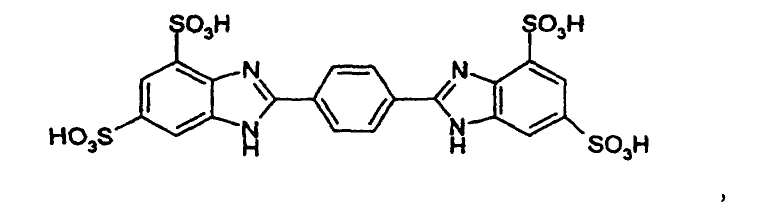
wobei das erste Filter und das zweite Filter in solchen Mengenanteilen vorliegen, dass hinsichtlich der vermittelten Lichtschutzfaktoren eine synergistische Wirkung eintritt.

2. Zusammensetzung nach Anspruch 1, **dadurch gekennzeichnet, dass** das Bis-hydroxyphenylbenzotriazol mit Kohlenwasserstoffgruppe in einer Konzentration von etwa 0,1 bis 15 Gew.-%, bezogen auf das Gesamtgewicht der Zusammensetzung, enthalten ist.

3. Zusammensetzung nach Anspruch 2, **dadurch gekennzeichnet, dass** das Bis-hydroxyphenylbenzotriazol mit Kohlenwasserstoffgruppe in einer Konzentration von etwa 0,2 bis 10 Gew.-%, bezogen auf das Gesamtgewicht der Zusammensetzung, vorliegt.

4. Zusammensetzung nach einem der Ansprüche 1 bis 3, **dadurch gekennzeichnet, dass** das Hydroxyphenylbenzotriazol mit Kohlenwasserstoffgruppe in Form von unlöslichen Partikeln vorliegt.

5. Zusammensetzung nach einem der Ansprüche 1 bis 4, **dadurch gekennzeichnet, dass** das Hydroxyphenylbenzotriazol mit Kohlenwasserstoffgruppe in mikronisierter unlöslicher Form vorliegt.

6. Zusammensetzung nach Anspruch 5, wobei das Bis-hydroxyphenylbenzotriazol mit Kohlenwasserstoffgruppe in mikronisierter unlöslicher Form eine mittlere Partikelgröße von 0,01 bis 2 µm aufweist.

7. Zusammensetzung nach Anspruch 6, wobei das Bis-hydroxyphenylbenzotriazol mit Kohlenwasserstoffgruppe in mikronisierter unlöslicher Form eine mittlere Partikelgröße von 0,02 bis 1,5 µm aufweist.

8. Zusammensetzung nach Anspruch 7, wobei das Bis-hydroxyphenylbenzotriazol in mikronisierter unlöslicher Form eine mittlere Partikelgröße von 0,05 bis 1,0 µm aufweist.

9. Zusammensetzung nach einem der Ansprüche 5 bis 9, **dadurch gekennzeichnet, dass** das Hydroxyphenylbenzotriazol mit Kohlenwasserstoffgruppe in mikronisierter unlöslicher Form durch Zerkleinerung eines Hydroxyphenylbenzotriaziazol mit Kohlenwasserstoffgruppe in Form von gröberen Partikeln in Gegenwart eines grenzflächenaktiven Stoffes erhältlich ist.

10. Zusammensetzung nach Anspruch 9, wobei der grenzflächenaktive Stoff unter den Alkylpolyglucosiden der Struktur CₙH₂ₙ₊₁ O(C₆H₁₀O₅)ₓH ausgewählt ist, wobei n eine ganze Zahl von 8 bis 16 bedeutet und x der mittlere Polymerisationsgrad der Einheit (C₆H₁₀O₅) ist und im Bereich von 1,4 bis 1,6 liegt.

11. Zusammensetzung nach Anspruch 10, wobei das grenzflächenaktive Alkylpolyglucosid durch Umsetzung einer C₁₋₁₂-Carbonsäure mit einer oder mehreren freien OH-Gruppen an der Glucosideinheit (C₆H₁₀O₅) erhältlich ist.

12. Zusammensetzung nach Anspruch 11, wobei die C₁₋₁₂-Carbonsäure unter Ameisensäure, Essigsäure, Propionsäure; Buttersäure, Sulfobemsteinsäure, Weinsäure oder Citronensäure ausgewählt ist.

13. Zusammensetzung nach einem der Ansprüche 9 bis 12, wobei der grenzflächenaktive Stoff in einer Konzentration von 1 bis 50 Gew.-%, bezogen auf das Hydroxyphenylbenzotriaziazol mit Kohlenwasserstoffgruppe in seiner mikronisierten Form verwendet wird.

14. Zusammensetzung nach einem der Ansprüche 1 bis 13, **dadurch gekennzeichnet, dass** die Verbindung mit Benzoazolylgruppen in einer Konzentration von 0,1 bis 15 Gew.-%, bezogen auf das Gesamtgewicht der Zusammensetzung, enthalten ist.

15. Zusammensetzung nach Anspruch 14, **dadurch gekennzeichnet, dass** die Konzentration im Bereich von 0,2 bis 10 Gew.-%, bezogen auf das Gesamtgewicht der Zusammensetzung, liegt.

16. Zusammensetzung nach einem der Ansprüche 1 bis 15, **dadurch gekennzeichnet, dass** der kosmetisch akzeptable Träger in Form einer Emulsion vom Öl-in-Wasser-Typ vorliegt.

17. Zusammensetzung nach einem der Ansprüche 1 bis 16, **dadurch gekennzeichnet, dass** sie ferner ein oder mehrere ergänzende, im UV-A- und/oder UV-B-Bereich wirksame, hydrophile oder lipophile Sonnenschutzfilter enthält, die von dem ersten und zweiten Filter verschieden sind.

18. Zusammensetzung nach Anspruch 16, **dadurch gekennzeichnet, dass** die zusätzlichen organischen Filter unter den Zimtsäurederivaten, Salicylsäurederivaten, Campherderivaten, Triazinderivaten, Benzimidazolderivaten, die von den in den vorhergehenden Ansprüchen definierten Derivaten verschieden sind, Benzophenonderivaten, Dibenzoylmethanderivaten, β,β-Diphenylacrylatderivaten, p-Aminobenzoesäurederivaten, Filterpolymeren und Filtersiliconen ausgewählt sind.

19. Zusammensetzung nach einem der Ansprüche 1 bis 18, **dadurch gekennzeichnet, dass** sie ferner als zusätzliche UV-Schutzmittel Pigmente oder Nanopigmente von Metalloxiden enthält, die gegebenenfalls umhüllt sind.

20. Zusammensetzung nach Anspruch 19, **dadurch gekennzeichnet, dass** die Pigmente oder Nanopigmente unter den Oxiden von Titan, Zink, Eisen, Zirconium oder Cer ausgewählt sind und gegebenenfalls umhüllt sind.

21. Zusammensetzung nach einem der Ansprüche 1 bis 20, **dadurch gekennzeichnet, dass** sie ferner mindestens einen Stoff zur Bräunung und/oder künstlichen Braunfärbung der Haut enthält.

22. Zusammensetzung nach einem der Ansprüche 1 bis 21, **dadurch gekennzeichnet, dass** sie ferner mindestens einen Zusatzstoff enthält, der unter den Fettsubstanzen, organischen Lösungsmitteln, Verdickungsmitteln, beruhigenden Stoffen, Antioxidantien, Trübungsmitteln, Stabilisatoren, Emollientien, Hydroxysäuren, Schaumverhütungsmitteln, Hydratisierungsmitteln, Vitaminen, Parfums, Konservierungsmitteln, grenzflächenaktiven Stoffen, Füllstoffen, Maslderungsmitteln, Polymeren, Treibmitteln, Alkalisierungsmitteln, Ansäuerungsmitteln oder Farbmitteln ausgewählt ist.

23. Zusammensetzung nach einem der Ansprüche 1 bis 22, **dadurch gekennzeichnet, dass** es sich um eine Zusammensetzung zum Schutz der menschlichen Epidermis oder eine Lichtschutzzusammensetzung handelt und **dadurch**, dass sie als nichtionische Vesikeldispersion, Emulsion und insbesondere Emulsion vom Öl-in-Wasser-Typ, Creme, Milch, Gel, Gelcreme, Suspension, Dispersion, Pulver, fester Stift, Schaum oder Spray vorliegt.

24. Zusammensetzung nach einem der Ansprüche 1 bis 22, **dadurch gekennzeichnet, dass** es sich um eine Zusammensetzung zum Schminken der Wimpern, der Augenbrauen oder der Haut handelt und sie in fester oder pastöser, wasserfreier oder wässriger Form als Emulsion, Suspension oder Dispersion vorliegt.

25. Zusammensetzung nach einem der Ansprüche 1 bis 22, **dadurch gekennzeichnet, dass** es sich um eine Zusammensetzung handelt, die für den Schutz der Haare gegen UV-Strahlung vorgesehen ist und **dadurch**, dass sie als Haarwaschmittel, Lotion, Gel, Emulsion oder nichtionische Vesikeldispersion vorliegt.

26. Verwendung der Zusammensetzung nach einem der Ansprüche 1 bis 22 für die Herstellung von kosmetischen Zusammensetzungen zum Schutz der Haut und/oder der Haare gegen UV-Strahlung und insbesondere gegenüber Sonnenlicht.

## Claims

1. Cosmetic composition for topical use, in particular for photo-protecting the skin and/or the hair, **characterized in that** it comprises, in a cosmetically acceptable support:
(a) as first screening agent, the bis-hydroxyphenylbenzotriazole hydrocarbon-based compound corresponding to the following formula: 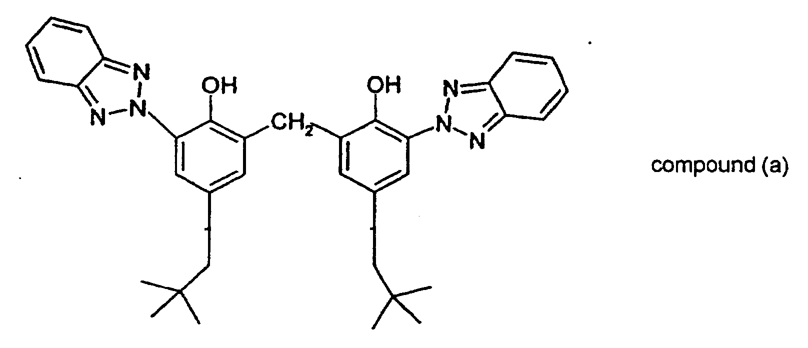
and (b) as second screening agent, the compound comprising at least two benzazolyl groups consisting of a salt of 1,4-bis(benzimidazolyl)phenylene-3,3',5,5'-tetrasulphonic acid having the following structure: 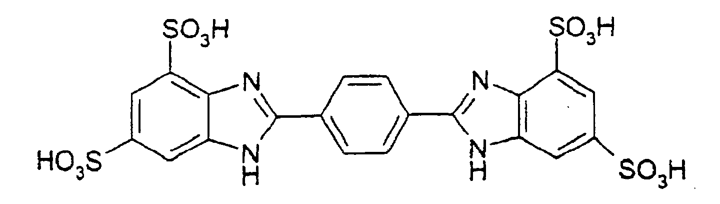
the said first and second screening agents being present in a proportion which produces synergistic activity as regards the sun protection factors imparted.

2. Composition according to Claim 1, **characterized in that** the bis-hydroxyphenylbenzotriazole hydrocarbon-based compound is present at a concentration ranging from 0.1 to 15% by weight approximately relative to the total weight of the composition.

3. Composition according to Claim 2, **characterized in that** the bis-hydroxyphenylbenzotriazole hydrocarbon-based compound is present at a concentration ranging from 0.2 to 10% by weight approximately relative to the total weight of the composition.

4. Composition according to any one of Claims 1 to 3, **characterized in that** the hydroxyphenylbenzotriazole hydrocarbon-based compound is in the form of insoluble particles.

5. Composition according to any one of Claims 1 to 4, **characterized in that** the hydroxyphenylbenzotriazole hydrocarbon-based compound is in insoluble micronized form.

6. Composition according to Claim 5, in which the bis-hydroxyphenylbenzotriazole hydrocarbon-based compound in insoluble micronized form has an average particle size ranging from 0.01 to 2 µm.

7. Composition according to Claim 6, in which the bis-hydroxyphenylbenzotriazole hydrocarbon-based compound in insoluble micronized form has an average particle size ranging from 0.02 to 1.5 µm.

8. Composition according to Claim 7, in which the bis-hydroxyphenylbenzotriazole hydrocarbon-based compound in insoluble micronized form has an average particle size ranging from 0.05 to 1.0 µm.

9. Composition according to any one of Claims 5 to 8, **characterized in that** the hydroxyphenylbenzotriazole hydrocarbon-based derivative(s) in insoluble micronized form can be obtained by a process of grinding a hydroxyphenylbenzotriazole hydrocarbon-based derivative in the form of particles of coarse size in the presence of a surfactant.

10. Composition according to Claim 9, in which the surfactant is chosen from alkylpolyglucosides of structure CₙH₂ₙ₊₁O(C₆H₁₀O₅)ₓH in which n is an integer from 8 to 16 and x is the average degree of polymerization of the unit (C₆H₁₀O₅) and ranges from 1.4 to 1.6.

11. Composition according to Claim 10, in which the alkylpolyglucoside surfactant can be obtained by reacting a C₁-C₁₂ carboxylic acid with one or more free OH functions on the glucoside unit (C₆H₁₀O₅).

12. Composition according to Claim 11, in which the said C₁-C₁₂ carboxylic acid is chosen from formic acid, acetic acid, propionic acid, butyric acid, sulphosuccinic acid, tartaric acid and citric acid.

13. Composition according to any one of Claims 9 to 12, **characterized in that** the surfactant is used at a concentration ranging from 1 to 50% by weight relative to the hydroxyphenylbenzotriazole hydrocarbon-based derivative screening agent in its micronized form.

14. Composition according to any one of Claims 1 to 13, **characterized in that** the compound containing benzazolyl groups is present at a concentration of between 0.1 and 15% by weight relative to the total weight of the composition.

15. Composition according to Claim 14, **characterized in that** the said concentration is between 0.2 and 10% by weight relative to the total weight of the composition.

16. Composition according to any one of Claims 1 to 15, **characterized in that** the said cosmetically acceptable support is in the form of an emulsion of oil-in-water type.

17. Composition according to any one of Claims 1 to 16, **characterized in that** it also comprises one or more additional hydrophilic or lipophilic sunscreens that are active in the UV-A and/or UV-B range, other than the said first and second screening agents.

18. Composition according to Claim 17, **characterized in that** the said additional organic screening agents are chosen from cinnamic derivatives, salicylic derivatives, camphor derivatives, triazine derivatives, benzimidazole derivatives other than those defined in the preceding claims, benzophenone derivatives, dibenzoylmethane derivatives, β,β'-diphenylacrylate derivatives, p-aminobenzoic acid derivatives, screening polymers and screening silicones.

19. Composition according to any one of Claims 1 to 18, **characterized in that** it also comprises, as additional UV photo-protective agents, pigments or nanopigments of coated or uncoated metal oxides.

20. Composition according to Claim 19, **characterized in that** the said pigments or nanopigments are chosen from titanium oxide, zinc oxide, iron oxide, zirconium oxide and cerium oxide, which are coated or uncoated.

21. Composition according to any one of Claims 1 to 20, **characterized in that** it also comprises at least one agent for artificially tanning and/or browning the skin.

22. Composition according to any one of Claims 1 to 21, **characterized in that** it also comprises at least one adjuvant chosen from fatty substances, organic solvents, thickeners, softeners, antioxidants, opacifiers, stabilizers, emollients, hydroxy acids, antifoaming agents, moisturizers, vitamins, fragrances, preserving agents, surfactants, fillers, sequestering agents, polymers, propellants, acidifying or basifying agents and dyes.

23. Composition according to any one of Claims 1 to 22, **characterized in that** it is a composition for protecting the human epidermis or an antisun composition and **in that** it is in the form of a nonionic vesicular dispersion, an emulsion, in particular an emulsion of oil-in-water type, a cream, a milk, a gel, a cream-gel, a suspension, a dispersion, a powder, a solid tube, a mousse or a spray.

24. Composition according to any one of Claims 1 to 22, **characterized in that** it is a make-up composition for the eyelashes, the eyebrows or the skin and **in that** it is in solid or pasty, anhydrous or aqueous form of an emulsion, a suspension or a dispersion.

25. Composition according to any one of Claims 1 to 22, **characterized in that** it is a composition intended for protecting the hair against ultraviolet rays and **in that** it is in the form of a shampoo, a lotion, a gel, an emulsion or a nonionic vesicular dispersion.

26. Use of the composition defined in any one of Claims 1 to 22, for the manufacture of cosmetic compositions for protecting the skin and/or the hair against ultraviolet radiation, in particular solar radiation.
